(19) Europäisches Patentamt

European Patent Office

Office européen des brevets

(11) EP 0 894 823 B1

(12) FASCICULE DE BREVET EUROPEEN

(45) Date de publication et mention
de la délivrance du brevet:
**11.09.2002 Bulletin 2002/37**

(51) Int Cl.⁷: **C08K 5/41**, A61L 33/00,
B01D 63/02

(21) Numéro de dépôt: **98420134.3**

(22) Date de dépôt: **29.07.1998**

(54) **Articles médicaux non cytotoxiques en polyuréthane**

Nicht zytotoxische medizinische Artikel aus Polyurethan

Not-cytotoxic medical articles from polyurethane

(84) Etats contractants désignés:
**AT BE CH DE ES FR GB IE IT LI NL SE**

(30) Priorité: **31.07.1997 FR 9710036**

(43) Date de publication de la demande:
**03.02.1999 Bulletin 1999/05**

(73) Titulaire: **HOSPAL INDUSTRIE**
**F-69883 Meyzieu Cédex (FR)**

(72) Inventeur: **Esposito, Guy**
**01700 Beynost (FR)**

(74) Mandataire: **Lejeune, Daniel**
**Gambro Patent Department**
**61, avenue Tony Garnier**
**69007 Lyon (FR)**

(56) Documents cités:
**EP-A- 0 145 614        FR-A- 2 268 058**
**US-A- 4 123 412        US-A- 4 879 032**
**US-A- 5 306 798**

## Description

L'invention a pour objet des articles médicaux non cytotoxiques après stérilisation ou désinfection par un procédé oxydant, à base de compositions génératrices de polyuréthane, et plus particulièrement des masses d'empotage pour échangeurs médicaux à membranes planes ou à fibres creuses. L'invention a également pour objet un procédé de préparation de ces articles médicaux en polyuréthane permettant de limiter fortement la cytotoxicité résultant de la stérilisation ou de la désinfection par un procédé oxydant tel que les rayonnements ionisants (irradiation gamma, faisceau d'électrons), les peroxydes gazeux (stérilisation dite par plasma froid), les peroxydes liquides ou tout autre procédé physique ou chimique faisant intervenir une réaction d'oxydation susceptible de dénaturer le matériau stérilisé.

Selon le cas, les masses d'empotage en polyuréthane sont destinées à former :

- une cloison cylindrique de séparation des deux compartiments d'un échangeur médical dont la membrane est constituée par un faisceau de fibres creuses. L'opération consistant à réaliser une telle cloison de séparation est usuellement désignée sous le vocable "d'empotage" ;
- ou un joint étanche dans un échangeur médical comprenant une membrane plane semi-perméable. L'opération consistant à réaliser un tel joint est usuellement désignée sous le vocable de "garnissage d'étanchéité". Néanmoins, afin de simplifier la présente description, cette opération sera également désignée sous le vocable "d'empotage".

Dans un souci de clarté de la présente description, le vocable de "joint" sera utilisé pour désigner indifféremment un joint étanche dans des échangeurs médicaux à membranes planes semi-perméables ou une cloison cylindrique de séparation dans des échangeurs médicaux dont la membrane est constituée par un faisceau de fibres creuses semi-perméables.

La présente invention est en particulier utile pour la fabrication des échangeurs pour des applications médicales sous la forme, par exemple, de dialyseurs, hémofiltres et oxygénateurs.

Il est de pratique courante de fabriquer des échangeurs pour des applications médicales en suivant les étapes générales qui suivent :

- préparer une membrane semi-perméable à partir d'une membrane plane ou conformer un faisceau de fibres creuses semi-perméables à partir de fibres creuses ;
- monter la membrane semi-perméable ou bien le faisceau de fibres creuses dans un boîtier et former, selon le cas, un joint étanche ou une cloison cylindrique de séparation des deux compartiments, à l'aide d'une composition adhésive génératrice de polyuréthane ;
- le cas échéant, fixer des embouts au boîtier, et
- stériliser l'appareil médical obtenu.

Les compositions adhésives génératrices de polyuréthane utilisées pour préparer un joint dans un échangeur médical, comprennent, d'une manière générale, avant polymérisation, un ou plusieurs polyisocyanates, un ou plusieurs polyols et, éventuellement, un ou plusieurs agents réticulants polyfonctionnels et/ou un ou plusieurs catalyseurs.

Le polyuréthane, une fois qu'il est durci, a pour fonction essentielle de former un joint étanche afin qu'il n'y ait pas d'infiltration entre les deux compartiments des échangeurs ou avec l'extérieur. Le risque d'infiltration doit, en particulier, être évité entre le compartiment à sang et le compartiment à dialysat des échangeurs médicaux pour le traitement du sang. Pour ce faire, la composition adhésive de polyuréthane doit présenter une adhérence satisfaisante avec les membranes semi-perméables des échangeurs, quelle que soit la nature chimique des matières qui les constituent. Egalement, cette composition doit présenter une adhérence satisfaisante avec les éléments des échangeurs avec lesquels elle est mise en contact, tels que le boîtier.

Une autre qualité importante exigée des échangeurs à usage biomédical est la biocompatibilité des masses d'empotage en polyuréthane, durcies et stérilisées, plus spécialement leur non cytotoxicité. Or, l'étape de stérilisation ou de désinfection par un procédé oxydant, surtout lorsqu'il s'agit d'une stérilisation par irradiation, peut rendre le polyuréthane cytotoxique.

Antérieurement, afin de former des masses d'empotage non cytotoxiques, diverses solutions ont été proposées, ainsi :

- dans le brevet américain US-4,332,927 il a été proposé des compositions génératrices de polyuréthane comprenant au moins un prépolymère à terminaisons isocyanato(-NCO), au moins un polyol et une quantité catalytique d'un composé d'étain dialcoyle dicarboxylé ;
- dans les brevets européens n°0393545 et n°0413265 et le brevet américain US-5,306,798, diverses compositions adhésives génératrices de polyuréthane à base de diphényl méthane diisocyanates (MDI) ou de dérivé de MDI, et de polyols spécifiques ont été proposées.

D'une manière différente et surprenante, la demanderesse a trouvé qu'il est possible de fabriquer des articles médicaux en polyuréthane stérilisés ou désinfectés par un procédé oxydant, qui sont non cytotoxiques, en particulier des masses d'empotage en polyuréthane, qui sont en outre suffisamment adhérentes avec les membranes semi-perméables des échangeurs mais non cytotoxiques après stérilisation ou désinfection par un procédé oxydant. Conformément à l'invention, on part d'une composition génératrice de polyuréthane contenant :

- au moins un polyisocyanate, de préférence non aromatique, à l'état de monomère ou de prépolymère ;
- au moins un polyol ; et
- au moins un composé dialkylsulfoxyde, de préférence à raison d'au moins 2 % en poids du poids total du ou des polyols.

Avantageusement, la composition génératrice de polyuréthane comprend en outre au moins un catalyseur de la réaction de polymérisation d'un polyisocyanate et d'un polyol.

La présente invention a également pour objet un procédé permettant de réduire la cytotoxicité des articles médicaux en polyuréthane, en particulier des masses d'empotage en polyuréthane, susceptible d'apparaître après stérilisation ou désinfection par un procédé oxydant, caractérisé en ce que l'on prépare la composition génératrice de polyuréthane à partir de :

- au moins un polyisocyanate, de préférence non aromatique, à l'état de monomère ou de prépolymère ;
- au moins un polyol, à l'état de monomère ou de prépolymère ;
- au moins un composé dialkylsulfoxyde, la quantité de composé dialkylsulfoxyde étant de préférence au moins égale à 2 % en poids rapporté au poids total des polyols ; et
- le cas échéant, au moins un catalyseur de la réaction de polymérisation d'un polyisocyanate et d'un polyol.

Dans le cadre de la présente invention, par articles médicaux en polyuréthane non cytotoxiques, on entend des articles conduisant à un pourcentage d'inhibition de croissance cellulaire (% ICG) fortement réduit grâce à la présence d'au moins un composé dialkylsulfoxyde. Ce pourcentage d'ICG est, de préférence, au plus égal à 30% en moyenne sur au moins 3 échantillons, quand les articles médicaux en polyuréthane sont soumis aux essais biologiques des matériaux et dispositifs médicaux et dentaires , partie 5 : méthodes in vitro, de la norme ISO 10-993, complétée par les conditions de mesure de la cytotoxicité utilisées par la demanderesse. Ces conditions spécifiques de mesure de la cytotoxicité sont énoncées ci-après, avec les exemples.

Une caractéristique essentielle de l'invention réside dans l'utilisation d'au moins un composé dialkylsulfoxyde pour préparer des articles médicaux en polyuréthane non cytotoxiques, plus spécialement des masses d'empotage destinées à enrober des échangeurs médicaux sous la forme de membranes planes ou de fibres creuses.

Les radicaux alkyles des composés dialkylsulfoxydes convenant à l'invention peuvent comporter un ou plusieurs groupements fonctionnels tels que les groupements hydroxy, mais sont exempts de groupements fonctionnels réagissant vivement avec un groupement isocyanato comme les groupements amines ou les groupements thiol.

Les composés dialkylsulfoxydes convenant à l'invention ont un point de fusion au plus égal à 40°C et ne doivent pas être toxiques pour les êtres humains dans le cas de diffusion dans le sang. Avantageusement, on choisit des composés dialkylsulfoxydes liquides à température ambiante, c'est-à-dire à une température de l'ordre de 20-25°C. Le composé dialkylsulfoxyde préféré est le diméthylsulfoxyde, plus connu sous l'abréviation "DMSO".

De préférence, la quantité de DMSO à prévoir pour atteindre des résultats satisfaisants au test de cytotoxicité (i.e. de préférence au plus 30% d'inhibition de croissance cellulaire en moyenne sur au moins 3 échantillons) est au moins égale à 2 % en poids du poids total du ou des polyols. Il est préférable que la quantité de DMSO n'excède pas 10 % en poids (rapporté au poids total des polyols), sinon un effet plastifiant conduisant à une diminution de la dureté peut se manifester dans le polyuréthane. De préférence encore, la quantité de DMSO est comprise entre 3% et 9 % en poids rapporté au poids total de polyols.

A titre d'exemples de polyols susceptibles de convenir à l'invention, on peut utiliser : l'huile de ricin ; les esters de polyol et de l'acide ricinoléique ; des polyéther polyols tels que le polyoxypropylène glycol et des polytétraméthylène éther glycols ; des homopolymères ou copolymères de butadiène porteurs d'au moins deux groupements hydroxy ; des esters de polyol et d'acide gras tel que l'huile de soja ou de l'huile de ricin, ou des esters de diacide, saturé ou non, et d'éthylène glycol comme l'adipate de polyéthylène glycol ; la N,N,N',N'-tétrakis (hydroxy-propyl) éthylènediamine ; des polycaprolactone polyols ; des oligomères polyols, en particulier des dimères polyols pouvant comprendre un groupement hydrocarboné, cyclique et saturé, et formés par condensation et réduction complète de deux acides gras insaturés ou formés par condensation, réduction partielle et estérification de deux acides gras insaturés ; des prépolymères polyols obtenus par réaction d'un excès de polyols avec un polyisocyanate, de préférence un polyisocyanate non aromatique ; des mélanges de deux ou plus des polyols précités.

Les polyisocyanates convenant à l'invention sont de préférence non aromatiques, c'est-à-dire exempts d'un ou plusieurs noyaux benzéniques, naphtalèniques, anthracéniques, etc. A titre d'exemples de polyisocyanates non aromatiques, à l'état de monomères ou de prépolymères, susceptibles de convenir à l'invention, on peut citer les polyisocyanates aliphatiques ou cycloaliphatiques, tels que le dicyclohexylméthyl-4,4'-diisocyanate (HMDI), l'isophorone-diisocyanate (IPDI) correspondant au 3-isocyanato méthyl-3,5,5-triméthylcyclohexylisocyanate, le triméthyl hexaméthylène diisocyanate, l'hexaméthylène diisocyanate (HDI) et ses dérivés de condensation tels que le biuret de HDI et l'isocyanurate de HDI. Des polyisocyanates aromatiques peuvent également être utilisés.

La quantité de polyisocyanate mise à réagir avec le polyol devra être suffisante pour fournir au moins un groupement isocyanato par groupement hydroxy de polyol. Un rapport en nombre NCO/OH égal ou supérieur à 1, de préférence égal ou supérieur à 1,1 est avantageux.

Bien entendu, les compositions de polyuréthane selon l'invention peuvent renfermer divers additifs classiquement utilisés dans le domaine technique en cause, tels que :

- au moins un catalyseur pour accélérer la réticulation, de préférence à raison de 0,01 à 2 % en poids de la composition totale. A titre d'exemple de catalyseur convenant à la présente invention, on peut citer les carboxylates d'étain tels que le dibutyl dilaurate d'étain (DBTL). La présence d'un catalyseur est recommandée lorsque les polyisocyanates sont non aromatiques ;

- au moins un promoteur d'adhésion pour conférer au polyuréthane durci une adhérence améliorée aux fibres creuses ou membranes planes semi-perméables, en particulier quand l'échangeur médical est constitué d'une membrane semi-perméable chargée négativement [i.e. une membrane contenant des charges négatives en excès détectable, en particulier par des mesures d'écoulement (potentiel Zéta)]. A titre de promoteur d'adhésion, on peut utiliser la polyamine de formule (I) décrite dans la demande de brevet européen n°0573310 ou une polyéthylèneimine (PEI). De préférence, on choisit une PEI de masse moléculaire moyenne en poids allant d'environ 600 à environ 10 000, plus aisée à mettre en oeuvre du fait de leur viscosité plus faible à température ambiante. La PEI peut être mise en oeuvre selon deux procédés différents (a) ou (b) qui suivent :

    a) la PEI peut servir à traiter la surface extérieure des membranes semi-perméables. Elle est alors appliquée sur la surface extérieure des fibres ou des canaux formés par les membranes semi-perméables planes ;
    b) la PEI peut être incorporée dans la composition adhésive génératrice de polyuréthane. Elle est alors mélangée avec l'un ou plusieurs des composants servants à préparer une composition adhésive génératrice de polyuréthane.

Un brevet représentatif de la technique de l'utilisation de PEI comme promoteur d'adhésion des masses d'empotage est la demande de brevet européen n°0710683.

Divers procédés de préparation des articles médicaux en polyuréthane selon l'invention peuvent être utilisés.

Conformément à un mode de réalisation préféré, on prépare une composition génératrice de polyuréthane à partir de deux composants qui sont conservés séparément, respectivement un premier composant constitué d'au moins un polyisocyanate, à l'état de monomère ou de prépolymère, et un second composant constitué d'au moins un polyol et d'au moins un composé dialkylsulfoxyde, de préférence à raison d'au moins 2 % en poids de composé dialkylsulfoxyde rapporté au poids total du ou des polyols et, le cas échéant, d'au moins un catalyseur et/ou d'au moins un promoteur d'adhésion. On mélange ces deux composants jusqu'à l'obtention d'un mélange homogène au moment de la fabrication de l'article médical tel qu'un joint étanche en polyuréthane.

Egalement, la composition génératrice de polyuréthane peut être présentée, avant utilisation, en trois composants, respectivement un premier composant constitué d'au moins un polyisocyanate, à l'état de monomère ou de prépolymère, un second composant constitué d'au moins un polyol et, selon le cas, d'au moins un catalyseur et/ou au moins un promoteur d'adhésion, et un troisième composant constitué d'au moins un composé dialkylsulfoxyde, de préférence à raison d'au moins 2 % en poids rapporté au poids du ou des polyols.

Les compositions adhésives génératrices de polyuréthane selon l'invention sont particulièrement bien adaptées aux membranes semi-perméables chargées négativement et conformées dans un seul type de matériau qui comprend notamment un homo- ou copolymère de l'acrylonitrile sous forme d'une membrane plane ou d'un faisceau de fibres creuses.

Un tel matériau, quand il est constitué d'un ou de plusieurs copolymères d'acrylonitrile, peut comprendre

    (1) un copolymère de l'acrylonitrile et d'au moins un monomère anionique ou anionisable, renfermant, le cas échéant, des unités provenant d'au moins un autre monomère à insaturation oléfinique capable d'être copolymérisé avec l'acrylonitrile, ou

(2) un copolymère de l'acrylonitrile et d'au moins un monomère anionique ou anionisable et d'au moins un monomère non ionique et non ionisable.

Certains de ces composés macromoléculaires, ainsi que les divers monomères susceptibles d'être retenus comme matières premières de leur fabrication, sont plus amplement décrits dans le brevet américain n°4,545,910 redélivré sous le n°Re.34239.

Parmi ces composés macromoléculaires, ceux avec lesquels les compositions adhésives génératrices de polyuréthane selon l'invention sont particulièrement bien adaptées sont définis sous (1) ci-avant, surtout quand la membrane semi-perméable est à l'état d'hydrogel. En particulier, l'invention convient particulièrement bien à ceux pour lesquels le comonomère anionique ou anionisable est oléfiniquement insaturé et porteur de groupements anioniques choisis parmi les groupes sulfonate, carboxyle, phosphate, phosphonate et sulfate, et plus particulièrement encore, quand ce comonomère est le méthallylsulfonate de sodium.

Bien entendu, la nature précise du contre-ion des groupements anioniques n'est pas essentielle au bon fonctionnement de l'invention.

Parmi les monomères à insaturation oléfinique capables d'être copolymérisés avec l'acrylonitrile, on peut citer les acrylates d'alkyle et, en particulier, l'acrylate de méthyle.

Les exemples ci-après illustrent l'invention sans aucunement en limiter la portée.

## EXEMPLES 1 ET 2

Préparation de polyuréthane (PUR) à partir d'une composition adhésive en deux composants, incorporant ou non un composé dialkylsulfoxyde.

Dans une cuve munie d'un agitateur, on introduit une quantité déterminée de polyol. On pèse une quantité déterminée de promoteur d'adhésion et on introduit cette quantité dans la cuve. Le cas échéant, on pèse une quantité déterminée de composé dialkylsulfoxyde et on introduit cette quantité à son tour dans la cuve. On agite alors, sous atmosphère d'azote ou d'air sec jusqu'à homogénéisation. Le mélange est ensuite dégazé sous vide, à température ambiante ou à chaud (maximum 40°C), avant son utilisation pour la préparation du polyuréthane par mélange avec de l'isocyanate, lui-même dégazé sous vide (pour éviter la présence de bulles).

Le taux de catalyseur est ajusté suivant le temps de gel désiré.

Pour environ la moitié de sa composition (premier composant), le PUR est à base d'une partie dite isocyanate.

L'autre partie de la composition servant à préparer le PUR (second composant) contient le mélange stable de polyol, de promoteur d'adhésion, de catalyseur et le cas échéant, de composé dialkylsulfoxyde, en l'occurrence le diméthylsulfoxyde (DMSO). Les proportions de la partie isocyanate et de la partie polyol sont calculées en fonction du poids équivalent de groupement isocyanato (NCO) et du poids équivalent de groupement hydroxy (OH) afin d'avoir un rapport NCO/OH égal à 1,1.

La nature chimique et la quantité des polyols, du polyisocyanate et du catalyseur des exemples 1 et 2 figurent dans le tableau ci-après.

| Nature chimique des composants | Exemple 1 | Exemple 2 |
|---|---|---|
| | Quantité de chaque composant (en % en poids) | |
| Composant isocyanate à base de prépolymère d'isocyanate aliphatique obtenu par réaction d'un excès de diisocyanate aliphatique avec du glycérol. | 49,3 | 49,3 |
| Composant polyol à base de polyesters de l'acide ricinoléique type ricinoléates de pentaérythritol. | 46,5 | 46,5 |
| Catalyseur d'organoétain. | 1,3 | 1,3 |
| Promoteur d'adhésion (1) | 2,9 | 2,9 |
| DMSO | 0 | 2,4 (2) |

(1) Il s'agit du produit commercial Ethoduomeen T-13 de AKZO Co.

(2) correspond à 4,9 parties en poids de DMSO pour 100 parties de composant polyol.

Empotage des fibres creuses

L'empotage avec une composition adhésive génératrice de polyuréthane ou PUR nécessite, au préalable, un séchage, au minimum, des extrémités du faisceau de fibres qui seront en contact avec le PUR.

Ensuite, on prépare la composition adhésive génératrice de polyuréthane en mélangeant les premier et second composants mentionnés au paragraphe précédent pour les besoins des exemples.

Aussitôt après, on coule la composition dans un réservoir muni de tubulures reliées aux deux extrémités d'un boîtier tubulaire où un faisceau de fibres creuses a été introduit et où il doit être empoté à ses deux extrémités. Préalablement à cette opération, le boîtier a été muni de bouchons à ses extrémités pour contenir la colle lors de l'empotage proprement dit.

On met en rotation le boîtier contenant le faisceau de fibres autour d'un axe perpendiculaire à l'axe longitudinal du faisceau et passant à mi-longueur du dispositif. Sous l'effet de la force centrifuge, la composition est déplacée aux extrémités du faisceau de fibres et vient enrober celles-ci. La composition pénètre également à l'intérieur des fibres mais cette pénétration est limitée par la compression de l'air prisonnier à l'intérieur des fibres. En outre, cette pénétration est maîtrisée en jouant sur deux paramètres : la force centrifuge (i.e. la vitesse de rotation du dispositif) et la température de l'air.

Après polymérisation de la composition, les bouchons sont enlevés et la masse d'empotage est tranché à un niveau au-delà de la pénétration de la composition dans les fibres de façon à ce que les fibres soient ouvertes pour permettre la circulation de fluide à l'intérieur des fibres.

Une fois l'assemblage terminé, le produit obtenu est ensaché hermétiquement pour être protégé de toute contamination microbiologique après stérilisation.

Le mode de stérilisation choisi est l'irradiation gamma à une dose d'irradiation au moins égale à 25 kGy garantissant une probabilité négligeable de contamination microbiologique après stérilisation.

Protocole de mesure de la cytotoxicité d'un polyuréthane (PUR).

Dans les exemples, on a mesuré la cytotoxicité des compositions adhésives de polyuréthane (PUR) durcies conformément aux recommandations de la norme ISO 10-993, partie 5, complétées de la façon suivante par la société HOSPAL :

A J1 (1er jour), dans des conditions aseptiques, une lignée de cellules fibroblastiques de souris (L929) est ensemencée à faible densité au fond de puits de culture ($\cong$ 5000 cellules par puits de 0,32 $cm^2$). Les cellules cultivées dans un milieu de culture additionné de 10 % de sérum de veau foetal (contenant des facteurs de croissance) adhèrent au plastique avant de se diviser.

A J2 (2ème jour), au moment où les cellules entrent en phase de croissance logarithmique, les cellules sont mises en contact avec l'éluat aqueux du PUR étudié, susceptible de contenir des substances extractibles.

Les conditions de préparation de l'éluat aqueux du PUR étudié, notamment les rapports surface/volume et température/durée sont décrites dans la norme ISO 10-993, partie 12.

L'éluat aqueux du PUR est dilué au demi avec un milieu de culture 2x (deux fois concentré). Une dilution de l'éluat au ¼ a aussi été testée après dilution au demi dans un milieu de culture 1x de la solution précédente.

A J5 (5ème jour), les puits de culture sont vidés, le tapis cellulaire est lavé et la densité et la viabilité des cellules sont quantifiées à l'aide d'une solution standardisée d'un colorant vital (le rouge neutre) capté par les cellules vivantes.

Environ trois heures après, le colorant en excès est éliminé par lavage et le colorant capté est extrait par un volume déterminé d'une solution d'acide acétique et d'éthanol.

La cytotoxicité est déterminée par rapport à un témoin de croissance absolue où le milieu de culture 2x a été dilué au demi avec de l'eau pour préparation injectable (i.e. de l'eau déminéralisée et bidistillée) dans lequel les cellules sont laissées cinq jours pour arriver à subconfluence, c'est-à-dire à forte densité.

Un témoin positif est systématiquement effectué à l'aide d'une substance toxique de référence ($HgCl_2$, ...). La préparation du témoin positif implique de diluer au demi un milieu de culture 2x avec de l'eau pour préparation injectable, d'additionner 6 $\mu$g/ml de $HgCl_2$ et de laisser les cellules cinq jours dans ce milieu dilué et toxique.

La cytotoxicité relative pour chaque éluat est exprimée par différence avec le témoin de croissance absolue en pourcentage d'inhibition de croissance cellulaire (% ICG).

La coloration de la solution d'acide acétique et d'éthanol est fonction de la concentration en cellules vivantes et est mesurée à l'aide d'un lecteur de plaques dans l'UV/visible, selon la procédure suivante :

$$\text{ICG (\%)} = 100 \, (D-d)/D$$

où

D représente la densité optique du témoin de croissance absolue,
d représente la densité optique de l'échantillon.

En outre, les résultats reportés dans le tableau ci-après correspondent à la moyenne de trois échantillons testés.

Dans le tableau ci-après sont reportés les résultats des mesures de cytotoxicité des PUR des exemples 1 et 2.

| Exemple n° | % ICG de l'éluat (37°C/120h - 12cm²/1ml) | |
|:---:|:---:|:---:|
| | Eluat dilué au 1/2 | Eluat dilué au 1/4 |
| 1 | 57 | 14 |
| 2 | 18 | 0 |

Au vu des résultats, il apparaît nettement que la présence du composé DMSO dans la formulation de PUR de l'exemple 2 conduit aux valeurs les plus faibles d'inhibition de croissance cellulaire (% ICG). Conformément à l'invention, le PUR de l'exemple 2 est estimé non cytotoxique.

## Revendications

1. Echangeur médical à membrane semi-perméable comprenant une masse d'empotage destinée à former une cloison cylindrique de séparation des deux compartiments de l'échangeur médical dont la membrane semi-perméable est constituée par un faisceau de fibres creuses, ou un joint étanche dans l'échangeur médical dont la membrane semi-perméable est plane, **caractérisé en ce que** la masse d'empotage est obtenue à partir d'une composition génératrice de polyuréthane comprenant :

   - un premier composant comprenant au moins un polyisocyanate à l'état de monomère ou de prépolymère ;
   - un second composant comprenant au moins un polyol, à l'état de monomère ou de prépolymère ;
   - et au moins un composé dialkylsulfoxyde.

2. Echangeur selon la revendication 1, **caractérisé en ce que** le polyisocyanate est non aromatique.

3. Echangeur selon la revendication 1, **caractérisé en ce que** le composé dialkylsulfoxyde représente au moins 2 % en poids rapporté au poids total du ou des polyols compris dans le second composant.

4. Echangeur selon la revendication 1, **caractérisé en ce que** la composition génératrice de polyuréthane comprend en outre un catalyseur de la réaction de polymérisation d'un polyisocyanate et d'un polyol.

5. Echangeur selon une des revendications 1 à 4, **caractérisé en ce que** le composé dialkylsulfoxyde a un point de fusion au plus égal à 40°C.

6. Echangeur selon la revendication 5, **caractérisé en ce que** le composé dialkylsulfoxyde est liquide à température ambiante.

7. Echangeur selon la revendication 6, **caractérisé en ce que** le composé dialkylsulfoxyde est le diméthylsulfoxyde.

8. Echangeur selon la revendication 7, **caractérisé en ce que** la quantité de diméthylsulfoxyde n'excède pas 10 % en poids rapporté au poids total du ou des polyols compris dans le second composant.

9. Echangeur selon la revendication 8, **caractérisé en ce que** la quantité de diméthylsulfoxyde est comprise entre 3 % et 9 % en poids rapporté au poids total du ou des polyols compris dans le second composant.

**10.** Procédé permettant de réduire la cytotoxicité des articles médicaux en polyuréthane susceptible d'apparaître après stérilisation ou désinfection par un procédé oxydant, **caractérisé en ce que** dans une composition génératrice de polyuréthane comprenant un premier composant comprenant au moins un polyisocyanate à l'état de monomère ou de prépolymère, un second composant comprenant au moins un polyol, on utilise au moins un composé dialkylsulfoxyde, à raison d'au moins 2 % en poids rapporté au poids total du ou des polyols compris dans le second composant.

**11.** Procédé selon la revendication 10, **caractérisé en ce que** le polyisocyanate est non aromatique.

**12.** Procédé selon la revendication 10 ou 11, **caractérisé en ce que** la composition génératrice de polyuréthane comprend en outre au moins un catalyseur de la réaction de polymérisation d'un polyisocyanate et d'un polyol.

**Patentansprüche**

**1.** Medizinischer Austauscher mit semipermeabler Membran, enthaltend eine Einbettmasse zur Bildung einer zylindrischen Wand zur Trennung der beiden Kompartimente eines medizinischen Austauschers, dessen semipermeable Membran durch ein Hohlfaserbündel gebildet wird, oder einer dichten Abdichtung in dem medizinischen Austauscher mit einer semipermeablen Flachmembran, **dadurch gekennzeichnet, daß** die Einbettmasse aus einer polyurethanbildenden Zusammensetzung, enthaltend:

- eine erste Komponente, die mindestens ein Polyisocyanat im Monomer- oder Präpolymerzustand enthält,

- eine zweite Komponente, die mindestens ein Polyol im Monomer- oder Präpolymerzustand enthält,

- und mindestens eine Dialkylsulfoxid-Verbindung,

erhalten wird.

**2.** Austauscher nach Anspruch 1, **dadurch gekennzeichnet, daß** das Polyisocyanat nicht aromatisch ist.

**3.** Austauscher nach Anspruch 1, **dadurch gekennzeichnet, daß** die Dialkylsulfoxid-Verbindung in einer Menge von mindestens 2 Gew.-%, bezogen auf das Gesamtgewicht des Polyols bzw. der Polyole in der zweiten Komponente, vorliegt.

**4.** Austauscher nach Anspruch 1, **dadurch gekennzeichnet, daß** die polyurethanbildende Zusammensetzung außerdem auch noch einen Katalysator für die Polymerisationsreaktion eines Polyisocyanats und eines Polyols enthält.

**5.** Austauscher nach einem der Ansprüche 1 bis 4, **dadurch gekennzeichnet, daß** die Dialkylsulfoxid-Verbindung einen Schmelzpunkt von höchstens 40°C aufweist.

**6.** Austauscher nach Anspruch 5, **dadurch gekennzeichnet, daß** die Dialkylsulfoxid-Verbindung bei Raumtemperatur flüssig ist.

**7.** Austauscher nach Anspruch 6, **dadurch gekennzeichnet, daß** es sich bei der Dialkylsulfoxid-Verbindung um Dimethylsulfoxid handelt.

**8.** Austauscher nach Anspruch 7, **dadurch gekennzeichnet, daß** das Dimethylsulfoxid in einer Menge von höchstens 10 Gew.-%, bezogen auf das Gesamtgewicht des Polyols bzw. der Polyole in der zweiten Komponente, vorliegt.

**9.** Austauscher nach Anspruch 8, **dadurch gekennzeichnet, daß** das Dimethylsulfoxid in einer Menge zwischen 3 und 9 Gew.-%, bezogen auf das Gesamtgewicht des Polyols bzw. der Polyole in der zweiten Komponente, vorliegt.

**10.** Verfahren zur Verringerung der Zytotoxizität von medizinischen Gegenständen aus Polyurethan, die nach Sterilisation oder Desinfektion durch einen oxidativen Prozeß auftreten kann, **dadurch gekennzeichnet, daß** man in einer polyurethanbildenden Zusammensetzung mit einer ersten Komponente, die mindestens ein Polyisocyanat im Monomer- oder Präpolymerzustand enthält, und einer zweiten Komponente, die mindestens ein Polyol enthält, mindestens eine Dialkylsulfoxid-Verbindung in einem Anteil von mindestens 2 Gew.-%, bezogen auf das Gesamtgewicht des Polyols bzw. der Polyole in der zweiten Komponente, einsetzt.

11. Verfahren nach Anspruch 10, **dadurch gekennzeichnet, daß** das Polyisocyanat nicht aromatisch ist.

12. Verfahren nach Anspruch 10 oder 11, **dadurch gekennzeichnet, daß** die polyurethanbildende Zusammensetzung außerdem mindestens einen Katalysator für die Polymerisationsreaktion eines Polyisocyanats und eines Polyols enthält.

**Claims**

1. Semi-permeable medical exchanger, comprising a potting body intended to form a cylindrical partition for sepa-rating the two compartments of a medical exchanger, the semi-permable membrane of which is composed of a bundle of hollow fibers, or a leaktight seal in the medical exchanger, comprising a semipermeable flat membrane, **characterized in that** the potting body is obtained from a polyurethane-generating composition comprising :

   •   one first component comprising at least one polyisocyanate, in the monomer or prepolymer state;
   •   one second component comprising at least one polyol, in the monomer or prepolymer state;
   •   and at least one dialkyl sulphoxide compound.

2. Exchanger according to claim 1, **characterized in that** the polyisocyanate is a non-aromatic.

3. Exchanger according to claim 1, **characterized in that** the dialkyl sulphoxide compound is at least equal to 2% by weight with respect to the total weight of the polyol or polyols comprised in the second component.

4. Exchanger according to Claim 1, **characterized in that** the polyurethane-generating composition additionally comprises a catalyst of the polymerization reaction of a polyisocyanate and of a polyol.

5. Exchanger according to one of Claims 1 to 4, **characterized in that** the dialkyl sulphoxide compound has a melting point at most equal to 40°C.

6. Exchanger according to Claim 5, **characterized in that** the dialkyl sulphoxide compound is liquid at ambiant temperature.

7. Exchanger according to Claim 6, **characterized in that** the dialkyl sulphoxide compound is dimethyl sulphoxide.

8. Exchanger according to claim 7, **characterized in that** the amount of dimethyl sulphoxide does not exceed 10 % by weight with respect to the total weight of the polyol or polyols comprised in the second component.

9. Exchanger according to claim 8, **characterized in that** the amount of dimethyl sulphoxide is between 3 % and 9 % by weight with respect to the total weight of the polyol or polyols comprised in the second component.

10. Process which makes it possible to reduce the cytotoxicity of polyurethane medical items liable to appear after sterilization or disinfection by an oxidizing process, **characterized in that**, in a polyurethane-generating composition comprising a first component comprising at least one polyisocyanate, in the monomer or prepolymer state, one second component comprising at least one polyol, use is made of at least one dialkyl sulphoxide compound in the proportion of at least 2% by weight with respect to the total weight of the polyol or polyols comprised in the second component.

11. Process according to claim 10 **characterized in that** the polyisocyanate is non aromatic.

12. Process according to claim 10 or 11, **characterized in that** the polyurethane-generating composition additionally comprises at least one catalyst of the polymerization reaction of a polyisocyanate and of a polyol.